# EUROPÄISCHE PATENTSCHRIFT

(11) **EP 0 676 942 B1**
(45) Veröffentlichungstag und Bekanntmachung des Hinweises auf die Patenterteilung: **23.10.1996**
(21) Anmeldenummer: 94901771.9
(22) Anmeldetag: 17.12.1993
(51) Int. Cl.: A61F 6/04

(54) **PROPHYLAKTIKUM**
CONTRACEPTIVE SHEATH
PRESERVATIF

(30) Priorität: 17.12.1992 DE 4242787
(43) Veröffentlichungstag der Anmeldung: 18.10.1995
(73) Patentinhaber: SCHOLL, Thomas, F-67000 Strasbourg (FR)
(72) Erfinder: SCHOLL, Thomas, F-67000 Strasbourg (FR)
(74) Vertreter: Söffge, Karen
(86) Internationale Anmeldenummer: DE9301214
(87) Internationale Veröffentlichungsnummer: WO9413231

(56) Entgegenhaltungen:
- CH-A- 513 644
- DE-A- 1 566 365
- DE-A- 2 202 462
- DE-A- 4 130 220
- US-A- 4 846 197
- US-A- 5 109 871

## Beschreibung

Die vorliegende Erfindung befaßt sich mit einem Verfahren zur Herstellung eines Prophylaktikums zur Empfängnisverhütung, insbesondere ein Prophylaktikum, das durch seine Formgebung einen sicheren Sitz auf dem erigierten Glied des Mannes gewährleistet, wie es beispielsweise aus der DE-OS 15 66 365 bekannt ist. Das im Stand der Technik bekannte Prophylaktikem offenbart an einer bestimmten Stelle im oberen Teil des Prophylaktikums einen elastischen Gummiring. der in eine Vertiefungsrille in der Tauchform von Hand oder maschinell eingelegt wird und dann das eigentliche Prophylaktikum auf die Tauchform aufgebracht wird. Das ringartige Gebilde kann sowohl auf der Außen- als auch auf der Innenseite einvulkanisiert werden.

Ein derartiger Ring wird beim Gebrauch als störend empfunden und ist darüberhinaus aufwendig und kostenintensiv in der Herstellung. Daher ist es Aufgabe der vorliegenden Erfindung, ein Verfahren zur Herstellung eines Prophylaktikums bereitzustellen, das einfach und kostengünstig in der Herstellung ist und den Anforderungen der Genehmigungsbehörden entspricht.

Diese Aufgabe wird durch die kennzeichnenden Merkmale des Hauptanspruchs gelöst.

Gemäß dem Anspruch 1 zeichnet sich das erfindungsgemäß Verfahren dadurch aus, daß die die einschnürende Formgebung des Tauchwerkzeugs in der Mitte durch eine Ringnut geprägt ist, in die das elastische Material des mindestens einen Tauchbades in mindestens einem Tauchvorgang in die Ringnut des Tauchwerkzeugs hineinfließt und dadurch eine integrierte größere Materialstärke an der Stelle der einschnürenden Formgebung bewirkt.

Vorteilhaft an dem erfindungsgemäßen Verfahren ist es, daß das elastische Material heim Tauchen in eine Ringnut am oberen Teil des Tauchwerkzeugs hineinfließt und die Ringnut eine geschlossene Kurve darstellt. Dadurch wird eine integrierte größere Materialstärke an der Stelle der einschnürenden Formgebung bewirkt.

Infolge der oben genannten Vorteile ergibt sich für den Anwender ein erheblicher Anwendungsvorteil, der das erfindungsgemäße Prophylaktikum angenehm im Gebrauch macht, ihm einen sicheren Sitz während des Gebrauchs verleiht und keine störenden Rauigkeiten, wie bei dem im Stand der Technik bekannten Prophylaktikum, an der Oberfläche aufweist.

Vorteilhaft erweist es sich, die einschnürende Formgebung in etwa der Form der Eichel des männlichen Gliedes anzupassen. Diese Form der Einschnürung ist jedoch nicht zwingend erforderlich, so daß auch jede andere beliebige polygone Form der Einschnürung gewählt werden kann, sofern sie in sich geschlossen ist.

Als weiterer Vorteil ist der Umstand anzusehen, daß der vordere Abschnitt des Prophylaktikums im Durchmesser größer als der hintere Abschnitt ist, so daß zwischen Eichel des männlichen Gliedes und Prophylaktikum ein gewisser Hohlräum gebildet wird.

Als besonders vorteilhaft bei dem Prophylaktikum, bei dem die einschnürende Formgebung der Form der Eichel des männlichen Gliedes angepaßt ist, erweist es sich, daß an einer vorbestimmten Stelle des aufgerollten Prophylaktikums eine Markierung angebracht ist, die dem Anwender die Lage der Formgebung anzeigt.

Weitere Vorteile und Ausgestaltungen sind den Unteransprüchen zu entnehmen.

Im nun folgenden wird die Erfindung anhand von Zeichnungen im Detail näher erläutert. Es eigen:
- Figur 1: die Seitenansicht eines erfindungsgemäßen Prophylaktikum, bei dem die Formgebung der Eichel des männlichen Gliedes angepaßt ist;
- Figur 2: die Seitenansicht eines erfindungsgemäßen Prophylaktikums, bei dem die Grenze zwischen vorderem und hinterem Teil kreisrund ist:

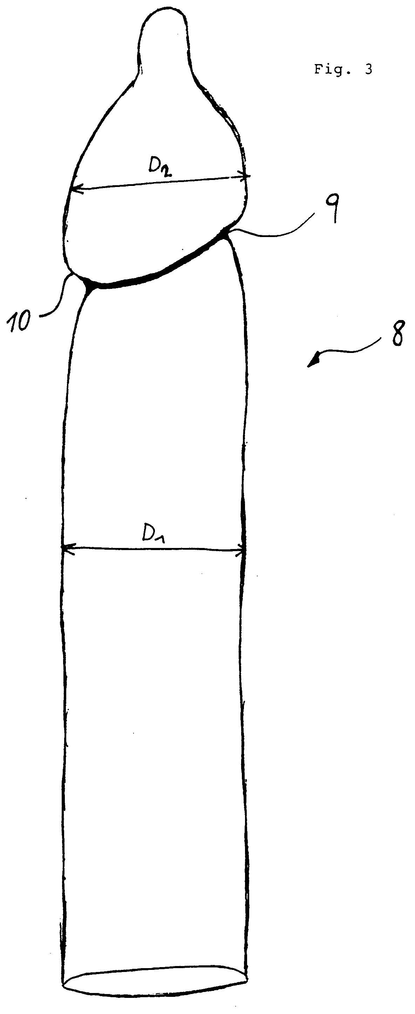

- Figur 3: eine Seitenansicht des Tauchwerkzeugs (8);
- Figur 4: einen vergrößerten Abschnitt des Tauchwerkzeugs (8).

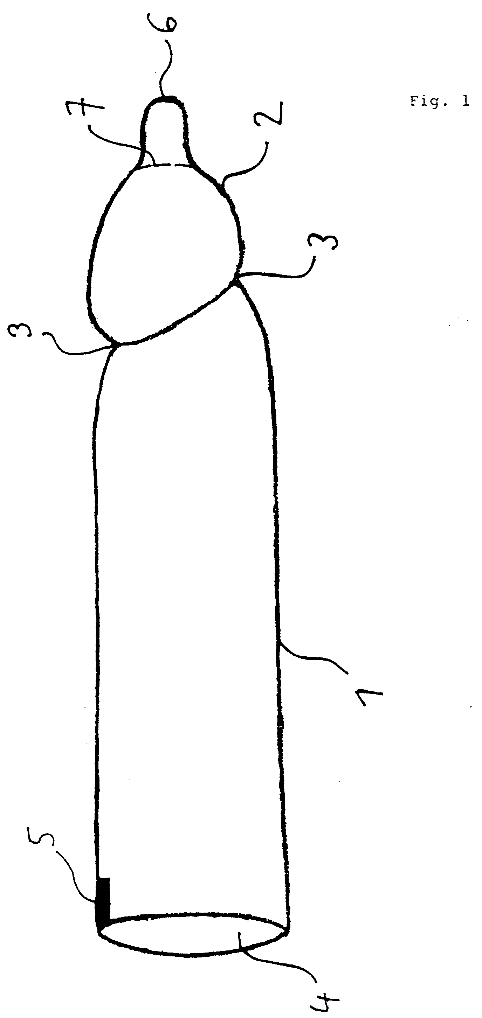

In Figur 1 wird ein gemäß dem erfindungsgemäßen Verfahren hergestelltes Prophylaktikum gezeigt, daß im wesentlichen aus zwei Abschnitten 1 und 2 besteht. Das eine Ende des Abschnitts 1 weist eine öffnung 4 auf, während das andere Ende durch die einschnürende Formgebung an der Grenze 3 endet. Im Anschluß an den Abschnitt 1 ist dar Abschnitt 2 angefügt. Erfindungsgemäß sind nun die Abschnitte 1 und 2 durch die Grenzlinie 3 abgegrenzt. Diese Grenzlinie 3 ist im Durchmesser etwas kleiner als der übrige langgestreckte zylindrische Abschnitt 1. Ebenso auch kleiner als der Durchmesser des vorderen Abschnitts 2. Dadurch ergibt sich zwangsläufig eine einschnürende Formgebung im vorderen Teil des langgestreckten zylindrischen Teils.

Entlang der Grenzlinie 3 ist verstärkt ein elastisches Material anzubringen, so daß dadurch ein fester sicherer Sitz des gesamten Prophylaktikums auf dem erigierten Glied des Mannes gewährleistet wird.

Durch die einschnürende Formgebung wird also das Prophylaktikum in zwei Abschnitte unterteilt und gleichzeitig an der Grenzlinie 3 verengt. Durch diese Verengung, die beispielsweise einen Durchmesser von 3 cm haben kann, wenn der Durchmesser des Abschnitts 1 bzw. des Abschnitts 2 3,5 cm aufweist. Diese Einschnürung setzt sich beim Geschlechtsverkehr hinter den Eichelrand des männlichen Gliedes und halt das gesamte Prophylaktikum in seiner ursprünglich aufgezogenen Lage. Dadurch wird ein Verrutschen der Penisvorhaut unter dem Prophylaktikum über die Eichel verhindert, was bisher als storender Einfluß beim Geschlechtsverkehr empfunden wurde.

Um die Lage der Formgebung der Grenzlinie 3 des aufgerollten Prophylaktikums mühelos zu erkennen, ist am Rande der Rolle eine Markierung angebracht, die im einfachsten Falle ein Farbstrich sein kann. Denkbar sind aber auch kleine Erhebungen im Material des Prophylaktikums.

Dadurch, daß der vordere Abschnitt 2 eine leichte Erweiterung erfahren kann, wird die Montage des Prophylaktikums über das Glied des Mannes weitgehend erleichtert.

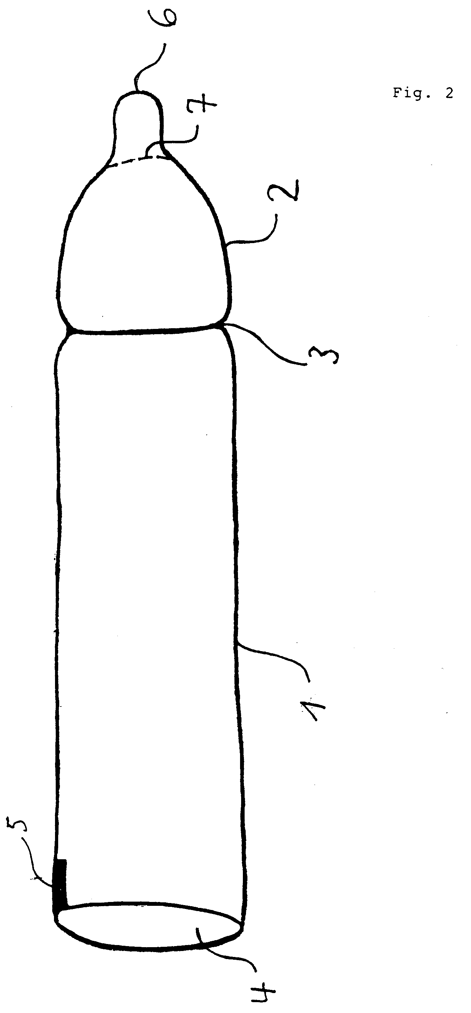

In Figur 2 wird eine weitere Variante des Prophylaktikums gezeigt. Diese unterscheidet sich jedoch nur darin, daß die einschnürende Grenzlinie 3 nicht der natürlichen Form der Eichel des männlichen Gliedes angepaßt ist, sondern eine kreisrunde Form annimmt, die ebenfalls durch elastisches Material verstärkt wird.

Der vordere Abschnitt 2 kann wahlweise durch eine Ausbuchtung 6 oder durch eine Rundung 7 abgeschlossen werden. Die übrigen Teile dieser erfindungsgemäßen Ausführung entsprechen denen der in Figur 1 beschriebenen.

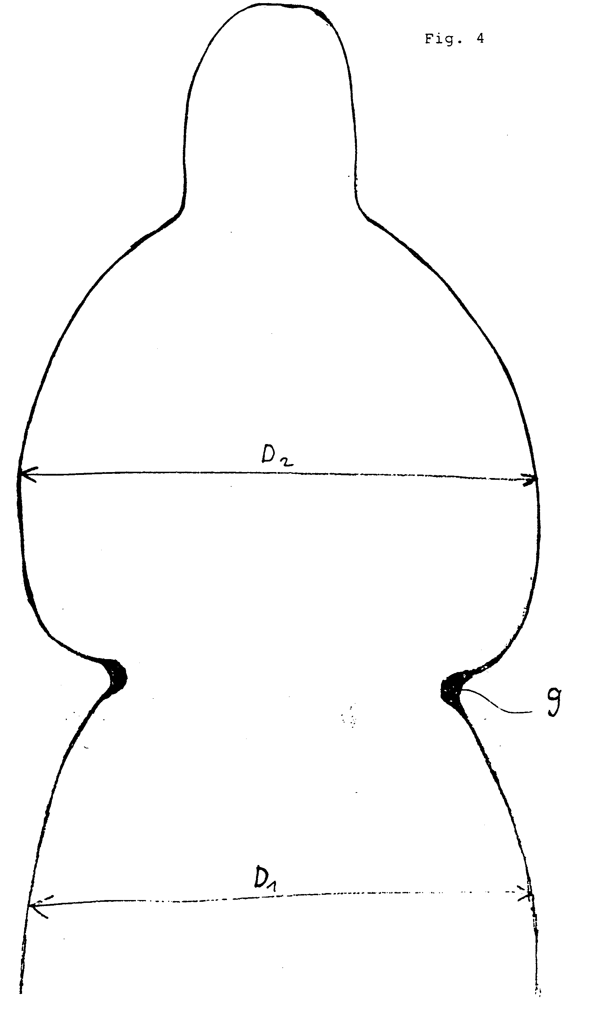

In Figur 4 ist ein bei dem erfindungsgemäßen Verfahren verwendetes Tauchwerkzeug 8 dargestellt, über das im an sich bekannten Tauchverfahren das elastische Material aufgebracht wird. Dieses Werkzeug 8 hat die spezielle Form des fertigen Produkts aus elastischem Material. Dabei ist der Durchmesser (D1) des ersten Abschnitts 1 kleiner als der Durchmesser (D2) des zweiten Abschnitts 2 am oberen Ende des Formgebungswerkzeugs. In der bereits sichtbaren Einschnürung 1 befindet sich eine Nut 9, die sich um den gesamten Schaft des Werkzeuge erstreckt. Die Nut 9 ist so ausgebildet, daß das Tauchmaterial während des Tauchvorganges gerade hierin verstärkt aufgebracht wird, so daß sich an dieser Stelle eine äußerlich glatte Verstärkung des elastischen Materials ergibt, wie es der Figur 4 zu entnehmen ist, wobei der dunkle Teil die Verstärkung des elastischen Materials darstellt.

## Patentansprüche

1. Verfahren zur Herstellung eines Prophylaktikums, bestehend aus einem dünnwandigen Formteil aus elastischem Material, dessen eines Ende geschlossen ist und dessen anderes Ende (4) offen ist, wobei sich an das offene Ende (4) ein zylindrischer, langgestreckter Teil anschließt, der im wesentlichen in zwei Abschnitte (1,2) unterteilt ist und eine einschnürende Formgebung im vorderen Teil des zylindrischen, langgestreckten Teils in der Nähe des geschlossenen Endes als Abschluß des vorderen Teils (2) aufweist, wobei die einschnürende Formgebung eine beliebige in sich geschlossene Kurve darstellt, mit einem bei einem Tauchvorgang die einschnürende Formgebung bewirkenden Tauchwerkzeug (8), dessen die einschnürende Formgebung prägende Form an ihrem tiefsten Punkt eine Ringnut (9) aufweist **dadurch gekennzeichnet**, daß die Ringnut so ausgebildet ist, daß in diese das elastische Material während des Tauchvorgangs hineinfließt und dadurch eine integrierte größere Materialstärke an der Stelle der einschnürenden Formgebung bewirkt.

2. Verfahren nach Anspruch 1, **dadurch gekennzeichnet,** daß die einschnürende Formgebung im Tauchwerkzeug (8) so ausgebildet wird, daß sich am Übergang vom vorderen (2) zum hinteren (1) Abschnitt beidseitig, innen und außen ein glatter Kurvenzug ergibt.

3. Verfahren nach Anspruch 1, **dadurch gekennzeichnet,** daß die einschnürende Formgebung in etwa der Form der Eichel des männlichen Gliedes angepaßt wird.

4. Verfahren nach Anspruch 1, **dadurch gekennzeichnet,** daß die einschnürende Formgebung kreisrund ist.

5. Verfahren nach Anspruch 1, **dadurch gekennzeichnet,** daß die Länge und der Durchmesser des vorderen (2) und hinteren (1) Abschnitts der Durchschnittslänge und dem Durchschnittsdurchmesser eines männlichen Gliedes angepaßt wird.

6. Verfahren nach Anspruch 1, **dadurch gekennzeichnet,** daß der vordere Abschnitt (2) im Durchmesser größer als der hintere Abschnitt (1) au gelegt wird.

7. Verfahren nach Anspruch 1, **dadurch gekennzeichnet,** daß der vordere und der hintere Abschnitt (1, 2) gleiche Durchmesser aufweisen.

8. Verfahren nach Anspruch 1, **dadurch gekennzeichnet,** daß der vordere geschlossene Abschnitt (2) am Ende mit einer Ausbuchtung (6) versehen wird.

## Claims

1. Procedure for the production of a contraceptive sheath, consisting of a thin-sided form part made of elastic material, of which the one end is closed and the other end (4) is open, whereby a cylindrical elongated part is connected to the open end (4), which is essentially subdivided in two sections (1, 2) and shows a constrictive forming in the front part of the cylindrical elongated part near the closed end as conclusion of the front part (2), whereby the constrictive forming shows an optional curve which is closed in itself, with an immersion tool (8) which effects the constrictive forming, whereby the constrictive forming is impressed at its deepest point by a ring groove (9), characterized in that the ring groove (9) is formed in a way that the elastic material flows into the ring groove (9) during the immersion process and thereby effects an integrated greater strength of the material in the place of the constrictive forming.

2. The procedure according to claim 1, characterized in that the constricted forming in the immersion tool (8) is made in a way that it results on the inner and outside in a smooth curve stream at the border from the front (2) to the end sections (1) on both sides.

3. The procedure according to claim 1, characterized in that the constricted forming is adapted to the form of the glans of the penis of a man.

4. The procedure according to claim 1 , characterized in that the constricted forming is circular.

5. The procedure according to claim 1 , characterized in that the length and the diameter of the front and end sections are adapted to the average diameter length and average diameter of the penis of a man.

6. The procedure according to claim 1 , characterized in that the front section (2) has a greater diameter than the end section (1).

7. The procedure according to claim 1 , characterized in that the front (2) and the end sections (1) show the same diameters.

8. The procedure according to claim 1 , characterized in that the front closed section (2) has a bulge at the end.

## Revendications

1. Procédure pour la fabrication de l'article prophylactique comprenant une partie à parois mince en matériau élastique, dont l'une des extrémités est fermée et l'autre (4) ouverte, à l'extrémité ouverte est attachée une partie allongée cylindrique, qui est effectivement subdivisée en deux sections (1, 2) et se présente à l'avant (2) de la partie allongée cylindrique près de l'extrémité fermée sous forme orifice serrée, qui termine la partie intérieur, cette orifice serré se détend ou se resserre à volonté et présente à l'aide d'un instrument d'immersion (8) une gorge annulaire (9) située à son extrémité inférieur, characterisée que cette gorge annulaire a une telle forme à ce que le matériau élastique glisse dans son intérieur pendant l'opération d'immersion, ce qui a comme conséquence un épaississement du matériau à l'emplacement de l'orifice serré.

2. Procédure selon revendication 1 caractérisée par une forme orifice serrée dans l' instrument d'immersion (8), qui résulte dans une courbe continuelle à l'intérieur et à l'extérieur à la transition de la section d'avant (2) à la section derrière (1).

3. Procédure selon revendication 1 caractérisée par une forme orifice serrée, qui s'adapte environ à la forme du gland de membre viril.

4. Procédure selon revendication 1 caractérisée par une forme orifice serrée d'être circulaire.

5. Procédure selon revendication 1 caractérisée par la longueur et le diamètre de la section d'avant (2) et derrière (1), qui sera adaptée à la longueur moyenne et le diamètre moyen d'un membre viril.

6. Procédure selon revendication 1 caractérisée par le diamètre de la section d'avant (2), qui est plus grande que de la section derrière (1).

7. Procédure selon revendication 1 caractérisée par la section d'avant et derrière (1, 2) ayant des diamètres pareils.

8. Procédure selon revendication 1 caractérisée par la section d'avant fermée (2), qui sera assortie d'une bombement (6).
